Europäisches Patentamt

European Patent Office

Office européen des brevets

(11) Publication number: **0 283 893**
**A1**

## EUROPEAN PATENT APPLICATION

(21) Application number: 88104021.6

(22) Date of filing: 14.03.88

(51) Int. Cl.⁴: **A61K 7/48**

(30) Priority: 23.03.87 IT 1979887

(43) Date of publication of application:
28.09.88 Bulletin 88/39

(84) Designated Contracting States:
**AT BE CH DE ES FR GB GR IT LI LU NL SE**

(71) Applicant: **R.P. DENIS S.p.A.**
**Via Leopardi 2**
**I-20123 Milan(IT)**

(72) Inventor: **Ravanelli, Giovanni**
**Via Cassanese 194**
**I-20090 Segrate Milan(IT)**

(74) Representative: **Giambrocono, Alfonso, Dr.**
**Ing. et al**
**Ing. A. Giambrocono & C. S.r.l. Via Rosolino**
**Pilo 19/B**
**I-20129 Milano(IT)**

(54) **Novel cosmetic formulations.**

(57) Novel, day and night, cosmetic formulations for the treatment of the skin are described, which contain a peculiar mixture of active ingredients selected from the group consisting of jojoba oil, hyaluronic acid, collagen, Retarderm, benzophenone and ribonucleic acid.

EP 0 283 893 A1

## Novel cosmetic formulations

The present invention concerns novel cosmetic formulations useful for the treatment of the skin.

More particularly, the object of the invention consists in a peculiar mixture of active ingredients, suitable for preparing formulations for cosmetics application. The formulations according to the invention, owing to their hydrating, nourishing and protective properties, can be employed either in the day or in the night treatment of the skin.

It was surprisingly found, as described in the following, that best results are achieved by suitably mixing some essential active ingredients selected from the group consisting of: jojoba oil, hyaluronic acid, collagen, Retarderm® (Petrolatum/Lanolin/SodiumPCA/Polysorbate 85), benzophenone 3 and ribonucleic acid. Ribonucleic acid is preferably employed in the night formulations, on account of its regenerative and energizing effect.

On the basis of the long cosmetologic experimental studies, the quantitative composition of the above-mentioned active ingredients in the formulations object of the invention which exhibits the best properties was discovered.

Said compositions of the active ingredients is as follows:

Jojoba oil 2.50 - 4.50% (weight)
Hyaluronic acid 0.04 - 0.06%
Collagen 0.50 - 1.50%
Retarderm® 1.50 - 3.50%
Benzophenone 3 0.10 - 0.30%
Ribonucleic acid 0.40 - 0.60%

To the active ingredients according to the invention preservatives, excipients, softenings, lubricants, emulsifiers, aromatizing and other agents, which are usually employed for this purpose, are added.

It was found that, in order to achieve the best cosmetic results, specific amounts of definite products are preferably added to the above-listed active ingredients. The invention, however, is not restricted to the formulations reported in the following, because the various additional products can be replaced with other, known equivalents.

Preferably, to the mixture of active ingredients of the invention the following additional products can be added according to the weight percentages set forth for two respective types of formulation:

FORMULATION 1 Decyl oleate 8.00 - 10.00%
Steareth 3.00 - 5.00%
Hydrogenated Polyisobutene 2.00 - 4.00%
Glyceryl stearate 1.50 - 2.50%
Petrolatum - lanolin oil 1.00 - 3.00%
Cetyl alcohol 0.50 - 1.50%
Dimethicone 0.30 - 0.80%
Polymethylmethacrylate 0.20 - 0.40%
Imidazolidinyl urea 0.20 - 0.40%
Propylparaben 0.10 - 0.20%
Methylparaben 0.10 - 0.20%
Triclosan 0.10 - 0.20%
Tridosium edetate 0.03 - 0.05%
BHA (butyl hydroxyanisole) 0.02 - 0.04%
Fragrance 0.10 - 0.30%
Deionized water up to 100,00%

FORMULATION 2 Glyceryl stearate 4.50 - 5.50%
Petrolatum/lanolin alcohol 3.50 - 4.50%
Octadedocanol 3.50 - 4.50%
Stearic acid 2.50 - 3.50%
Methyl Gluceth 2.50 - 3.50%
Stearyl heptanoate 1.50 - 2.50%

Ceteareth 0.50 - 1.50%
Cetyl phosphate 0.50 - 1.50%
Dimethicone 0.30 - 0.80%
Imidazolidinyl urea 0.20 - 0.50%
Triethanolamine 0.10 - 0.30%
Propylparaben 0.10 - 0.20%
Methylparaben 0.10 - 0.20%
Triclosan 0.10 - 0.20%
Quaternium 15 0.01 - 0.03%
BHA (butyl hydroxyanisole) 0.02 - 0.04%
Fragrance 0.10 - 0.30%
Deionized water up to 100%

It was particularly found that, for preparing formulations for the day treatment of the skin, the best results are obtained when the Formulation 1 is added, according to the above-mentioned amounts, to the jojoba oil, hyaluronic acid, collagen, Retarderm® and benzophenone 3 active ingredients. Formulation 2, on the contrary, affords best results fot the night treatment of the skin if added to all the above-mentioned active ingredients, including ribonucleic acid.

It was surprisingly found that the formulations according to the invention are highly effective, their action consisting not only in a mere absorption by the skin, but in that the skin itself is put in the best conditions for reacting, depending and reproducing itself, thus achieving the optimum conditions for both prevention and a normal cosmetic treatment.

More particularly, the day-formulation exerts a hydrating and hardening action and the night-formulation energizes and is effective against the wrinkle formation, by re-establishing the hydration and elasticity equilibriums of the cutaneous tissues.

The cosmetic formulations of the invention are prepared preferably as creams or emulsions by knwon mixing techniques.

The hydrating and hardening effects of the creams of the invention were experimentally verified for two inventive creams, a night-cream A and a day-cream B respectively, having the following compositions (in weight percent):

Cream A (night)Jojoba oil 4.00%
Hyaluronic acid 0.05%
Collagen 0.50%
Retarderm® 3.00%
Benzophenone 3 0.20%
Ribonucleic acid 0.50%
Glyceryl stearate 5.00%
Petrolatum/lanolin alcohol 4.00%
Octadodecanol 4.00%
Stearic acid 3.00%
Methyl Gluceth 3.00%
Stearyl heptanoate 2.00%
Ceteareth 1.00%
Cetyl phosphate 1.00%
Dimethicone 0.50%
Imidazolidinyl urea 0.30%
Triethanolamine 0.20%
Propylparaben 0.15%
Methylparaben 0.15%
Triclosan 0.15%
Quaternium 15 0.02%
BHA (butyl hydroxyanisole) 0.03%
Fragrance 0.20%
Deionized water up to 100%

Cream B (day)Jojoba oil 3.00%
Hyaluronic acid 0.04%
Collagen 1.00%
Retarderm® 2.00%
Benzophenone 3 0.20%
Decyl oleate 9.00%
Steareth 4.00%
Hydrogenated Polyisobutene 3.00%
Glyceryl stearate 2.20%
Petrolatum - lanolin oil 2.00%
Cetyl alcohol 1.00%
Dimethicone 0.50%
Polymethylmethacrylate 0.30%
Imidazolidinyl urea 0.30%
Propylparaben 0.15%
Methylparaben 0.15%
Triclosan 0.15%
Tridosium edetate 0.04%
BHA (butyl hydroxyanisole) 0.03%
Fragrance 0.20%
Deionized water up to 100,00%

Hydrating activityCream A and cream B were evaluated for hydrating activity according to the impedance metric "in vivo" method described in the literature (L. Bernardi, Boll. Soc. It. Biologia Sperim. 58, 1982, p. 353).

The method consists in evaluating the hydrating effects by measuring the electrical parameter $R_o$, which is inversely proportional to the wetting degree of the horny layer. The two creams were applied to a group of 28 female subjects, in good health conditions, between 17 and 49 years old.

1 ml of cream A (night) were applied, every night for 14 consecutive nights, to 25 $cm^2$ area of the left forearm. Measurements were carried out in basal conditions, i.e. before the application of the product and on the fifteenth day (prolonged test). Cream B (day) was applied on the palmar face of the right forearm, in the same quantity and on the some skin area as above, taking into account the basal measurement and the measurement after two hours from the application (short-term test). The evaluations of the effects at different times (after two hours or on the fifteenth day) were suggested by the different formulations of products A and B and their different application purposes.

The results showed a significant impedance ($R_o$) decrease in both cases, which confirms the different effects produced by the two creams according to the purposes for which they were individually formulated.

The day cream has the functional purpose of hydrating the skin and the aesthetic necessity not to make it greasy: it also presents formulation characteristics allowing the fast hydration of the skin, said effects lasting for a number of hours. The night-cream, owing to its formulation characteristics, can act for a greater number of hours and, with the contribution of a lipid costituent which is better tolerated during the night, forms a more occluding hydrolipidic film, thus retaining in the epidermis the water of perspiratio insensibilis. By means of such properties, the day-cream B is effective in a shorter time, while the night-cream A is more effective for a long-term hydration.

Hardening activity

The hardening effect of cream A and cream B was evaluated by extensometric measurements on the skin carried out by an extensometer.

The instrument consists of a mechanical part controlled by an electronic console. Two metal plates are applied to the skin and driven by an engine at constant force. When the applied force is the same, the more the two plates depart from each other the more loose is the skin. To this corresponds a higher reading. Day and night creams were applied on the forearms of 59 female subjects, between 18 and 45 years old.

Measurements were carried out as basal and after 20 days of treatment.

The day-cream was employed for 26 subjects and the night-cream for 28. The statistically significant results are reported in the following table, both creams showing tonic and hardening activities on the skin, with a more evident tonic activity of the day-cream.

ESTENSOMETRY

| Day-cream | | Night-cream | |
|---|---|---|---|
| Basal | 20 days | Basal | 20 days |
| 76 | 71 | 99 | 51 |
| 77 | 75 | 78 | 78 |
| 92 | 59 | 88 | 79 |
| 89 | 71 | 104 | 78 |
| 116 | 87 | 73 | 61 |
| 72 | 70 | 73 | 69 |
| 87 | 81 | 72 | 70 |
| 96 | 75 | 77 | 72 |
| 90 | 81 | 74 | 74 |
| 96 | 78 | 93 | 90 |
| 88 | 75 | 95 | 68 |
| 103 | 80 | 98 | 77 |
| 71 | 70 | 101 | 97 |
| 78 | 55 | 82 | 85 |
| 83 | 62 | 97 | 79 |
| 93 | 73 | 95 | 88 |
| 108 | 80 | 84 | 84 |
| 79 | 62 | 76 | 68 |
| 81 | 76 | 83 | 75 |
| 95 | 60 | 107 | 82 |
| 87 | 67 | 99 | 91 |
| 99 | 80 | 76 | 75 |
| 95 | 91 | 96 | 85 |
| 72 | 72 | 88 | 70 |
| 94 | 68 | 73 | 55 |
| 74 | 59 | 102 | 84 |

| Day-cream | | Night-cream | |
|---|---|---|---|
| Basal | 20 days | Basal | 20 days |
| | | 105 | 81 |
| | | 82 | 69 |

With mere illustrative purposes are reported some preparative examples of the formulations according to the invention.

## EXAMPLE 1

### Preparation of Day-cream (B)

40 mg of hyaluronic acid are dissolved in 70 ml of deionized water under stirring at room temperature (step $A_1$). Separately, 400 mg of trisodium edetate, 150 mg of methylparaben and 300 mg of imidazolidinyl urea are dissolved in about 630 ml of water and the solution is heated to 75°C (step $A_2$).

The following fat materials are separately blended, while increasing the temperature to 75°C (step B):

Decyl oleate 9.0 g
Steareth 4.0 g
Hydrogenated Polyisobutene 3.0 g
Jojoba oil 3.0 g
Glyceryl stearate 2.2 g
Petrolatum/lanolin oil 2.0 g
Retarderm® 2.0 g
Cetyl alcohol 1.0 g
Dimethicone 500 mg
Benzophenone 3 200 mg
Propylparaben 150 mg
Triclosan 150 mg
BHA 30 g

The above-mentioned phases $A_2$ and B are mixed in a planetary mixer, allowed to cool, under vacuum and with a continued stirring, while controlling the temperature.

At 50°C, the phase $A_1$ is added to the mixture and cooling is continued until 30°C. At this point a drop of collagen, 200 ml of perfume and then 300 mg of polymethylmethacrylate are added.

Mixing of the cream is continued until homogeneous.

## EXAMPLE 2

### Preparation of the night-cream (A)

The procedure is the same of exampre 1, except that 50 mg of hyaluronic acid is used in phase $A_1$.

Phase 2 consists of 300 mg of imidazolidinyl urea, 200 mg of triethanolamine, 150 mg of methylparaben and 20 mg of quaternium 15. The remaining materials, forming the phase B and the final phase, listed in the above-mentioned formulation of cream A, are added according to the respective percent ratios.

## Claims

1. Cosmetic day and night formulations for the treatment of the skin, comprising preservatives, excipients, softening, lubricants, emulsifiers, aromatising agents, characterized in that they contain a mixture of active ingredients selected from the group consisting of jojoba oil, hyaluronic acid, Collagen, Retarderm, Benzophenone 3 and ribonucleic acid.

2. Cosmetic day formulation for the treatment of the skin according to claim 1, characterized in that the mixture of active ingredients consists of:

Jojoba oil 2.50 - 4.50%
Hyaluronic acid 0.04 - 0.06%
Collagen 0.50 - 1.50%
Retarderm® 1.50 - 3.50%
Benzophenone 3 0.10 - 0.30%

3. Cosmetic night formulation for the treatment of the skin according to claim 1, characterized in that the mixture of active ingredients consists of:

Jojoba oil 2.50 - 4.50%
Hyaluronic acid 0.04 - 0.06%
Collagen 0.50 - 1.50%
Retarderm® 1.50 - 3.50%
Benzophenone 3 0.10 - 0.30%
Ribonucleic acid 0.40 - 0.60%

# EUROPEAN SEARCH REPORT

European Patent Office

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | PATENT ABSTRACTS OF JAPAN, unexamined applications, field C, vol. 11, no. 3, January 7, 1987 THE PATENT OFFICE JAPANESE GOVERNMENT page 18 C 395 * Kokai-no. 61-180 705 (SHISEIDO CO.) * -- | 1 | A 61 K 7/48 |
| X | GB - A - 1 283 892 (I.I.LUBOWE) * Page 1, lines 10-12,46-55; page 2, lines 72-78 * -- | 1 | |
| X | FR - A - 2 574 661 (ROUSSEL-UCLAF) * Examples; page 4, lines 12-14 * -- | 1 | |
| X | EP - A1 - 0 191 128 (TERAD) * Examples 3,4,6 * -- | 1 | **TECHNICAL FIELDS SEARCHED (Int. Cl.4)** A 61 K 7/00 |
| A | EP - A1 - 0 180 505 (CLARINS) * Page 5, lines 5-12; claim 3 * -- | 1-3 | |
| A | FR - A - 2 052 143 (ACADEMIE SCIENTIFIQUE DE BEAUTE) * Claim 1 * -- | 1-3 | |
| A | DE - A1 - 3 512 717 (KAO CORP.) * Page 5, lines 19-23 * ---- | 1-3 | |

The present search report has been drawn up for all claims

| Place of search | Date of completion of the search | Examiner |
|---|---|---|
| VIENNA | 30-06-1988 | IRMLER |